(19) Europäisches Patentamt
European Patent Office
Office européen des brevets

(11) Publication number: **0 105 534**
**B1**

# (12) EUROPEAN PATENT SPECIFICATION

(45) Date of publication of patent specification: **08.10.86**

(21) Application number: **83200912.0**

(22) Date of filing: **20.06.83**

(51) Int. Cl.$^4$: **C 07 D 233/64,**
C 07 D 249/08,
C 07 D 231/12,
A 61 K 31/415, A 61 K 31/41

(54) **Substituted heterocyclylphenylsulphonyl and phosphonyl amidines, process for their preparation and their pharmaceutical use.**

(30) Priority: **29.07.82 IT 2263782**

(43) Date of publication of application:
**18.04.84 Bulletin 84/16**

(45) Publication of the grant of the patent:
**08.10.86 Bulletin 86/41**

(84) Designated Contracting States:
**AT BE CH DE FR GB IT LI LU NL SE**

(56) References cited:
**EP-A-0 053 407**
**US-A-4 020 083**
**US-A-4 239 896**

(73) Proprietor: **ISTITUTO DE ANGELI S.p.A.**
**Via Serio, 15**
**I-20139 Milano (IT)**

(72) Inventor: **Cereda, Enzo**
**Via Romagnolo 2A**
**I-15057 Tortona (Allessandria) (IT)**
Inventor: **Donetti, Arturo**
**Viale Romagna 4**
**I-20133 Milan (IT)**
Inventor: **Giachetti, Antonio**
**Via Guerrini 3**
**I-20133 Milan (IT)**
Inventor: **Del Soldato, Piero**
**Via E. Toti 22**
**I-20052 Monza (Milan) (IT)**

(74) Representative: **Appoloni, Romano et al**
**ING. BARZANO' & ZANARDO MILANO S.p.A.**
**Via Borgonuovo 10**
**I-20121 Milano (IT)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 105 534 B1

Courier Press, Leamington Spa, England.

**Description**

The present invention relates to a new substituted heterocyclylphenylsulphonyl and phosphonylamidines, to process for their preparation and to pharmaceutical compositions containing them. The new compounds are useful as metabolic intermediates susceptible of conversion, by oral biotransformation, to antagonist compounds acting at histamine H-2 receptors.

It is known that classical antihistamines, such as mepyramine, are capable to antagonize some effects of histamine mediated by $H_1$-rèceptors. However, these compounds have no effect on gastric acid secretion which is instead affected by other antihistaminic agents defined by Black *et al.* (Nature *236,* 385, 1972) as antihistamine $H_2$- receptor antagonists. This has indicated that another kind of receptors ($H_2$) already preconized by Ash and Shild (Brit. J. Pharmacol. Chem. Ther., *27,* 427—39, 1966) is involved in the gastric secretory response which is not blocked by the conventional antihistamines of the $H_1$-type.

Examples of $H_2$-receptor antagonists capable of antagonizing gastric acid secretion include burimamide, metiamide, and cimetidine. The clinical efficacy of the latter as a gastric antisectretory agent stimulated a search for agents with higher potency, longer duration of action, and lower side effects.

Recently, new $H_2$-antagonists, such as ranitidine (Bradshaw, *et al.,* Brit. J. Pharmacol. *66,* 464P, 1979), tiotidine (P. O. Jellin, Life Sci. *25,* 2001, 1979) and BL 6341 (Cavanagh, et al., Fed. Proc., *40,* 2652, 1981) have been described.

In a structural sense these compounds resemble cimetidine, since they contain a substituted heterocycle joined by a methylthioethyl side chain to a "urea equivalent" neutral polar group. In our European Patent Applications 53407 and 94747 we have described a new class of histamine $H_2$-antagonists, namely heterocyclylphenylformamidines, which are potent $H_2$-blockers and active antagonists of gastric acid secretion. These new compounds do not resemble the so far known $H_2$-antagonists, and are characterized by a substituted phenylformamidino groupment bearing variously substituted heterocyclic rings.

Object of the present invention are heterocyclylphenylsulphonyl and phosphonylamidines having the following general formula (I)

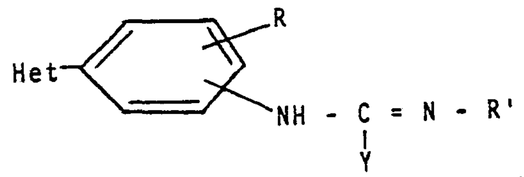

(I)

in which Het represents unsaturated five-membered heterocyclic ring containing from 2 to 3 nitrogen atoms, R is a hydrogen atom, a lower alkyl or alkoxy group containing from 1 to 3 carbon atoms or a halogen atom, Y represents a group of formula $—SO_3H$ or of formula

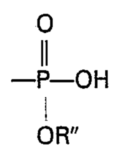

(where R″ is a lower alkyl group containing from 1 to 3 carbon atoms), R′ represents a linear or branched $C_1$—$C_8$ alkyl group which may contain a hetero atom such as sulfur, an oxygen or nitrogen atom, a linear or branched $C_3$—$C_5$ alkenyl group, a $C_3$—$C_7$ cycloalkyl or alkyl cycloalkyl group, phenyl group (optionally substituted by a lower alkyl or alkoxy group containing from 1 to 3 carbon atoms or a halogen atom), unsubstituted benzyl when Het is imidazole, R is hydrogen and Y is a sulphonic or phosphonic group; tautomers thereof and acid addition salts of the aforesaid compounds.

For pharmaceutical use the acid addition salts referred to the above will be physiologically compatible acid addition salts. The term "acid addition salts" includes salts with inorganic or organic acids.

Suitable physiologically compatible acid addition salts include, for example, salts formed with hydrochloric, maleic, fumaric, citric, tartaric or sulphuric acid.

It is to be understood that although the double bond in the amidine radical has been inserted in a particular position, other tautomeric forms are possible, and that different tautomeric forms are also possible in the heterocyclic ring. It is also to be understood that the sulphonyl- or phosphonylamidine radical may be in its zwitterionic form. The present invention includes such tautomeric forms within its scope, both in terms of the compounds of the invention and in terms of the manufacturing process.

In the compounds of formula (I) Het represents a heterocyclic ring containing from 2 to 3 nitrogen atom which is an unsaturated five-member ring, e.g. imidazole, triazole, pyrazole ring; when R represents a halogen atom it may be a chlorine atom; when R′ represents a linear or branched alkyl group it is an alkyl group containing from 1 to 8 carbon atoms which may contain an oxygen atom, e.g. hydroxypropyl, hydroxybutyl, methoxypropyl group, a sulfur atom, e.g. methylthioethyl, ethylthioethyl group, a nitrogen

atom, e.g. cyanoethyl group; when R′ represents a linear or branched alkenyl group it is an alkenyl group containing from 3 to 5 carbon atoms; when R′ represents a cycloalkyl or alkylcycloalkyl group it contains from 3 to 7 carbon atoms; when R′ represents an aryl or aralkyl group the aromatic ring is a phenyl (optionally substituted by a lower alkyl or alkoxy group containing from 1 to 3 carbon atoms or by a halogen atom which may be a chlorine atom).

In the above mentioned formula (I) the sulphonyl- or phosphonylamidine radical may be in the ortho meta or para position of the benzene ring with respect to the Het group, and the group R is in any position of the benzene ring.

Preferred compounds according to the present invention include those wherein the sulphonyl or phosphonylamidine radical is in the para position with respect to the heterocyclic ring, Het represents 2-imidazolyl, 4-imidazolyl, 3-pyrazolyl or 3-(1,2,4-triazolyl) group, R represents a hydrogen atom, a methyl, a methoxy group or a chlorine atom, Y is a group of formula $—SO_3H$ of of formula

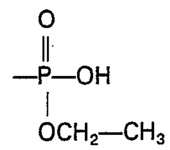

R′ represents a linear or branched alkyl or alkenyl group containing from 3 to 5 carbon atom (optionally containing a sulfur or an oxygen atom or a nitrogen in a cyano group) and their physiologically compatible acid addition salts. Such compounds generally have better activity and are therefore preferred as antisecretory-antiulcer agents and for the treatment of disorders of the gastrointestinal tract.

According to a further feature of the invention there is provided a process for the preparation of the compounds of general formula (I) which comprises reacting a heterocyclylphenylamine of formula (II)

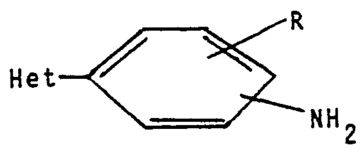

(II)

in which Het and R are as above described, with a compound of formula

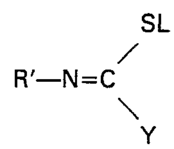

(III)

in which R′ and Y are as above described and L is a lower alkyl group containing from 1 to 3 carbon atom, for example methyl.

The reaction is generally carried out at a temperature from 0° to 60°C, preferably at room temperature in the presence of a polar inert organic solvent such as dioxane or acetonitrile.

The intermediate compounds of formula (III), in which Y represents a sulphonic acid group, can be produced by known methods for example by reacting an alkali thioxo methan sulphonate of formula (IV)

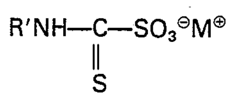

(IV)

in which R′ is as hereinbefore defined and M represents an alkali metal, for example sodium or potassium, with an organic alkyl iodide or dimethyl sulphate in refluxing acetonitrile.

The intermediate compounds of formula (III) wherein Y represents a phosphonic acid monoalkylester can be prepared by known methods, for example by reacting a thiocarbamoyl phosphonate of formula (V)

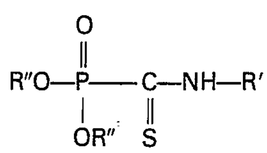

(V)

in which R′ and R″ are as above described, with an organic iodide of formula LJ, where L is as above defined, for example methyl iodide in the presence or in the absence of a polar inert solvent such as dioxane or acetonitrile.

The compounds of general formula (I) prepared according to the above process can also be converted with organic or inorganic acids into physiologically compatible acid addition salts as hereinbefore defined, for example by conventional methods such as by reacting the compounds as bases with the solution of the corresponding acids in a suitable solvent. Particularly preferred acids include, for example, hydrochloric, sulphuric, maleic or fumaric acid. The salts obtained are generally soluble in water.

The compounds of the present invention do not possess antihistamine ($H_2$) activity "per se" as they were found inactive in the "in vitro" assays usually employed for $H_2$ antagonist activity (e.g. the inhibition of the positive chronotropic effect of histamine in isolated guinea pig atria).

However, the compounds show antihistamine ($H_2$) activity when administered by oral route to rats. To demonstrate their "in vivo" antihistamine effect rats were used in which, activation of the histamine ($H_2$) receptors in the gastrointestinal tract by the specific agonist dimaprit (100 mg/kg intravenously) causes the appearance of severe gastric lesion (P. Del Soldato, Pharmacol. Res. Comm. *14,* 175, 1982). Compounds were adminstered to female fasting (24 hrs) Sprague-Dawley rats at variable intervals prior the challenge with dimaprit. The gastric lesions were evaluated 60 min after the injection of the agonist.

The protection of the ulcers by means of compounds, expressed as a percentage of animals with no signs of gastric damage, indicates antihistamine ($H_2$) effect.

The results of a typical experiment concerning the classical $H_2$ antagonist cimetidine and two of the compounds object of the present invention are reported in the following table:

| Compounds of | Dose mg/kg p.o. | Dimaprit Induced Gastric Ulcer % Protection at 1 hr | 6 hr |
|---|---|---|---|
| Example 3 | 1 | 30 | 40 |
| | 3 | 30 | 70 |
| Example 7 | 1 | 30 | 50 |
| | 3 | 40 | 60 |
| Cimetidine | 20 | 80 | 0 |
| | 80 | 100 | 10 |

According to a further feature of the present invention there are provided pharmaceutical compositions comprising as active ingredient at least one compound of formula (I), as hereinbefore defined, or a physiologically compatible acid addition salt thereof in association with a pharmaceutical carrier or excipient. For pharmaceutical administration the compounds of general formula (I) and their physiologically compatible acid addition salts may be incorporated into the conventional pharmaceutical preparations in either solid or liquid form. The compositions may, for example, be presented in a form suitable for oral or parenteral administration. Preferred forms include for example, capsules, tablets, coated tablets, ampoules or vials containing lyophilized mass of the salt.

The active ingredient may be incorporated in excipients or carriers conventionally used in pharmaceutical compositions such as for example talc, gum arabic, lactose, gelatine, magnesium stearate, corn starch, aqueous or non-aqueous vehicles, polyvinylpirrolidone, mannitol.

The compositions are advantageously formulated at dosage unit, each dosage unit being adapted to supply a fixed dose of the active ingredient. Each dosage unit may conveniently contain from 10 mg to 500 mg and preferably from 50 mg to 250 mg.

The following examples illustrate the present invention without limiting it.

Example 1

1-Methylthio-1-isopropylimino-methanesulphonate

The title compound was prepared according to the method of W. Walter and C. Rohloff [*Liebis Ann. Chem.* 491 (1977)] by refluxing for two hours a solution of 1-isopropylamino-1-thioxo-sodium methanesulphonate (30.8 g) and dimethylsulphate (37.8 g) in acetonitrile (650 ml). The compound which precipitated on standing was collected by filtration to give a white solid. M.p. 155°C dec.

Similarly the following intermediates were obtained:

1-Methylthio-1-ethylimino-methanesulphonate. White solid, M.p. 148—149°C.

1-Methylthio-1-allylimino-methanesulphonate. Waxy solid.

1-Methylthio-1-methylthioethylimino-methanesulphonate. Thick oil.

Example 2

S-Methyl-N-Isopropyl-thioimidoyl-phosphonic acid monoethyl ester

A solution of diethyl-N-isopropyl-thiocarbamoyl phosphonate (11 g), prepared according to V. A. Petrov and A. A. Neimysheva, *Zhur. Obsch. Khimii 29,* 1819 (1959) and methyliodide (48 ml) was refluxed for two hours and then evaporated to dryness. By crystallizing the crude residue from acetonitrile and diethylether, the title compound was obtained as a white solid. M.p. 94—97°C dec.

In a similar manner the following intermediates were also produced:

S-Methyl-N-ethyl-thioimidoyl phosphonic acid monoethyl ester. Thick oil.

S-Methyl-N-methyl-thioethylthioimidoyl phosphonic acid monoethyl ester. Thick reddish oil.

Example 3

N-Isopropyl-N′-[4-(imidazol-4-yl)phenyl]-amidino sulphonic acid

A solution of 1-methylthio-1-isopropyliminomethanesulphonate (6.8 g) in acetonitrile (150 ml) was added slowly to a solution of 4-(4-amino phenyl)-1-H-imidazole (5 g) in acetonitrile (250 ml) at room temperature. The reaction mixture was stirred overnight and the product which crystallized was collected by filtration to give the crude title compound. This was purified through its maleate salt in 95% ethanol. M.p. 209°C dec.

Analysis

| | | | | |
|---|---|---|---|---|
| $C_{17}H_2N_4O_7S$ | Found % | C 47.87 | H 4.82 | N 13.11 |
| | Calc. % | C 48.10 | H 4.75 | N 13.20 |

Example 4

N-Allyl-N′-[4-(imidazol-4-yl)-phenyl]-amidino sulphonic acid

A solution of 1-methylthio-1-allyl-iminomethanesulphonate (6.13 g) in acetonitrile (50 ml) was added slowly to a suspension of 4-(4-amino-phenyl)-1-H-imidazole (5 g) in acetonitrile (60 ml). The reaction mixture was stirred two days at room temperature, cooled and filtered. The crude title compound was purified through its maleate salt from 95% ethanol. M.p. 183—184°C.

Analysis

| | | | | |
|---|---|---|---|---|
| $C_{17}H_{18}N_4O_7S$ | Found % | C 47.92 | H 4.36 | N 13.17 |
| | Calc. % | C 48.34 | H 4.29 | N 13.26 |

Example 5

N-Ethyl-N′-[4-(imidazol-4-yl)-phenyl]-amidino sulphonic acid

A solution of 1-methylthio-1-ethyl-imino methanesulphonate (0.7 g) in acetonitrile (10 ml) was dropped into a suspension of 4-(4-amino-phenyl)-1-H-imidazole, monohydrochoride (0.6 g) in acetonitrile (30 ml). The reaction mixture was stirred three days at room temperature and then the white solid still in suspension was collected by filtration. This crude compound was triturated with methanol to give the title compound as its hydrochloride salt. M.p. 198—200°C dec.

Analysis

| | | | | |
|---|---|---|---|---|
| $C_{12}H_{15}N_4SO_3Cl$ | Found % | C 43.51 | H 4.62 | N 16.85 |
| | Calc. % | C 43.57 | H 4.57 | N 16.94 |

Example 6

By utilizing the suitable 1-methylthio-1-substituted imino methane-sulphonates, all prepared according to Example 1, the substitution of the following phenylamines:

a) 4-(3-methyl-4-amino phenyl)-1-H-imidazole
b) 4-(3-methoxy-4-amino phenyl)-1-H-imidazole
c) 4-(3-chloro-4-amino phenyl)-1-H-imidazole
d) 4-(3-amino phenyl)-1-H-imidazole
e) 2-(4-amino phenyl)-1-H-imidazole
f) 3-(4-amino phenyl)-pyrazole
g) 3-(4-amino phenyl)-1,2,4-triazole

for the 4-(4-amino phenyl)-1-H-imadazole in the procedure of Examples 3, 4 and 5, leads to the production of the following compounds:

N-Methylthioethyl-N′-[4-(imidazol-4-yl)-phenyl]-amidino sulphonic acid
N-Methoxyethyl-N′-[4-(imidazol-4-yl)-phenyl]-amidino sulphonic acid
N-Cyanoethyl-N′-[4-(imidazol-4-yl)-phenyl]-amidino sulphonic acid
N-n-Octyl-N′-[4-(imidazol-4-yl)-phenyl]-amidino sulphonic acid
N-Cyclohexyl-N′-[4-(imidazol-4-yl)-phenyl]-amidino sulphonic acid
N-4-Chlorophenyl-N′-[4-(imidazol-4-yl)-phenyl]-amidino sulphonic acid
N-Benzyl-N′-[4-(imidazol-4-yl)-phenyl]-amidino sulphonic acid

N-Ethylthioethyl-N'-[4-(imidazol-4-yl)-2-methyl phenyl]-amidino sulphonic acid
N-n-Butyl-N'-[4-(imidazol-4-yl)-2-methoxy phenyl]-amidino sulphonic acid
N-3-Methyl-but-2-enyl-N'-[4-(imidazol)-2-chlorophenyl]-amidino sulphonic acid
N-Isopropyl-N'-[3-(imidazol-4-yl)-phenyl]-amidino sulphonic acid
N-Cyclopropylmethyl-N'-[4-(imidazol-2-yl)-phenyl]-amidino sulphonic acid
N-Phenyl-N'-[4-(pyrazol-3-yl)-phenyl]-amidino sulphonic acid
N-Methoxyethyl-N'-[4-(1,2,4-triazol-3-yl)-phenyl]-amidino sulphonic acid

Example 7

N-Isopropyl-N'-[4-(imidazol-4-yl)-phenyl]-amidino phosphonic acid, monoethyl ester

A solution of 4-(4-amino phenyl)-1-H-imidazole (3.85 g) and S-methyl-N-isopropyl-thioimidoyl phosphonic acid monoethyl ester (6 g) in acetonitrile (140 ml) was stirred overnight at room temperature. The solid product which separated out was filtered and recrystallized from water and acetone to give the title compound as a base. M.p. 235°C.

Analysis

| | | | | | | | |
|---|---|---|---|---|---|---|---|
| $C_{15}H_{21}N_4O_3P$ | Found % | C | 53.07 | H | 6.19 | N | 16.47 |
| | Calc. % | C | 53.56 | H | 6.29 | N | 16.66 |

Example 8

N-Methylthioethyl-N'-[4-(imidazol-4-yl)-phenyl]-amidino phosphonic acid monoethyl ester

A solution of 4-(4-amino phenyl)-1-H-imidazole (5 g) and S-methyl-N-methylthioethyl-thioimidoyl phosphonic acid monoethyl ester (8.1 g) in acetonitrile (200 ml) was stirred overnight at room temperature. The solid product which separated on standing was filtered and recrystallized from acetone to give the title compound as a base. M.p. 240—241°C.

Analysis

| | | | | | | | |
|---|---|---|---|---|---|---|---|
| $C_{13}H_{21}N_4O_3PS$ | Found % | C | 45.16 | H | 6.17 | N | 16.12 |
| | Calc. % | C | 45.38 | H | 6.15 | N | 16.27 |

Example 9

Following the procedure of Example 7 and 8 and utilizing the suitable S-methyl-thioimidoyl phosphonic acid monoethyl ester, when instead of 4-(4-amino phenyl)-1-H-imidazole, there was used each of the following phenyl amines:

a) 4-(3-methyl-4-amino phenyl)-1-H-imidazole
b) 4-(3-methoxy-4-amino phenyl)-1-H-imidazole
c) 4-(3-chloro-4-amino phenyl)-1-H-imidazole
d) 4-(3-amino phenyl)-1-H-imidazole
e) 2-(4-amino phenyl)-1-H-imidazole
f) 3-(4-amino phenyl)-pyrazole
g) 3-(4-amino phenyl)-1,2,4-triazole

there were obtained monoethyl esters of these amidino phosphonic acids:

N-Benzyl-N'-[4-(imidazol-4-yl)-phenyl]-amidino phosphonic acid, monoethyl ester
N-3-Methoxy phenyl-N'-[4-(imidazol-4-yl)-phenyl]-amidino phosphonic acid, monoethyl ester
N-Cyclopropylmethyl-N'-[4-(imidazol-4-yl)-phenyl]-amidino phosphonic acid, monoethyl ester
N-Allyl-N'-[4-(imidazol-4-yl)-phenyl]-amidino phosphonic acid, monoethyl ester
N-n-Octyl-N'-[4-(imidazol-4-yl)-phenyl]-amidino phosphonic acid, monoethyl ester
N-Cyanoethyl-N'-[4-(imidazol-4-yl)-phenyl]-amidino phosphonic acid, monoethyl ester
N-Methylthioethyl-N'-[4-(imidazol-4-yl)-2-methyl phenyl]-amidino phosphonic acid, monoethyl ester
N-Methoxy-N'-[4-(imidazol-4-yl)-2-chlorophenyl]-amidino phosphonic acid, monoethyl ester
N-Isopropyl-N'-[4-(imidazol-4-yl)-2-methoxy phenyl]-amidino phosphonic acid, monoethyl ester
N-Cyclohexyl-N'-[3-(imidazol-4-yl)-phenyl]-amidino phosphonic acid, monoethyl ester
N-Cyanoethyl-N'-[4-(imidazol-2-yl)-phenyl]-amidino phosphonic acid, monoethyl ester
N-Phenyl-N'-[4-(pyrazol-3-yl)-phenyl]-amidino phosphonic acid, monoethyl ester
N-3-Methyl-but-2-enyl-N'-[4-(1,2,4-triazol-3-yl)-phenyl]-amidino phosphonic acid, monoethyl ester

The following examples of pharmaceutical compositions according to the invention are reported:

Example 10

| Tablets | |
|---|---|
| active ingredient | 150 mg |
| lactose | 250 mg |
| corn starch | 30 mg |
| magnesium stearate | 3 mg |

Method of preparation: the active ingredient, lactose, and corn starch were mixed and homogeneously moistened with water. After screening of the moist mass and drying in a tray driver, the mixture was again passed through a screen and magnesium stearate was added. Then the mixture was pressed into tablets weighing 433 mg each. Each tablet contains 150 mg of active ingredient.

Example 11

Capsules

| | |
|---|---|
| active ingredient | 150 mg |
| corn starch | 50 mg |
| magnesium stearate | 2 mg |

Method of preparation: the active ingredient was mixed with the auxiliary products, and the mixture was passed through a screen and mixed homogeneously in a suitable device. The resulting mixture was filled into hard gelatine capsule (202 mg per capsule); each capsule contains 150 mg of active ingredient.

Example 12

Vials

| | |
|---|---|
| active ingredient | 150 mg |
| water for injection q.s. | 5 ml |

Method of preparation: the active ingredient was dissolved in the water in appropriate amounts, and then the resulting solution was introduced into 5 ml vials under sterile conditions. Each vial contains 150 mg of active ingredient.

**Claims for the Contracting States: BE CH DE FR GB IT LI LU NL SE**

1. Compounds of general formula (I)

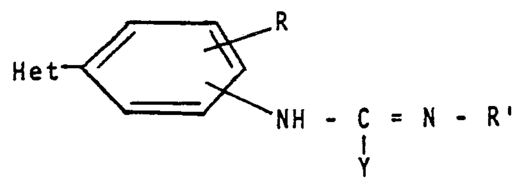

(I)

wherein Het represents an unsaturated five-membered heterocyclic ring containing from 2 to 3 nitrogen atoms, R is a hydrogen atom, a lower alkyl or alkoxy group containing from 1 to 3 carbon atoms or a halogen atom, Y represents a group of formula $—SO_3H$ or of formula

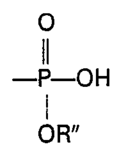

(wherein R″ is a lower alkyl group containing from 1 to 3 carbon atoms), R′ represents a linear or branched $C_1—C_8$ alkyl group which may contain a hetero-atom such as sulfur, an oxygen or nitrogen atom, a linear or branched $C_3—C_5$ alkenyl group, a $C_3—C_7$ cycloalkyl or alkyl cycloalkyl group, phenyl group (optionally substituted by a lower alkyl or alkoxy group containing from 1 to 3 carbon atoms or a halogen atom), unsubstituted benzyl when Het is imidazole, R is hydrogen and Y is a sulphonic or phosphonic group; tautomers thereof and acid addition salts of the aforesaid compound.

2. Physiologically compatible acid addition salts of compounds of formula (I) as claimed in claim 1.

3. Salts as claimed in claim 2 formed with hydrochloric, maleic, sulphuric or fumaric acid.

4. Compounds as claimed in any one of the preceding claims wherein the sulphonyl or phosphonylamidine radical is in the para position of the benzene ring with respect to the Het group.

5. Compounds as claimed in any one of the preceding claims wherein Het represents 2-imidazolyl, 4-imidazolyl, 3-pyrazolyl or 3-(1,2,4-triazolyl) group, R represents a hydrogen atom, a methyl, a methoxy group or a chlorine atom, Y is a group of formula $—SO_3H$ or of formula

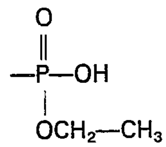 ,

R′ represents a linear or branched alkyl or alkenyl group containing from 3 to 5 carbon atom (optionally containing a sulfur or an oxygen atom or a nitrogen in a cyano group).

6. Physiologically compatible acid addition salts of compounds as claimed in claim 5.

7. Maleic, hydrochloric, sulphuric or fumaric acid addition salts of compounds as claimed in claim 5.

8. A process for the preparation of compounds of general formula (I) as claimed in claim 1 which comprises reacting a compound of formula (III)

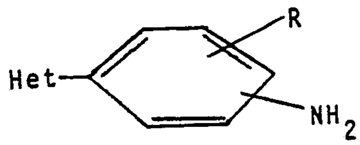

(II)

in which Het and R are as defined in claim 1, with a compound of formula (III)

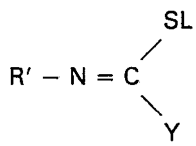

(III)

in which R′ and Y are as defined in claim 1 and L represents a lower alkyl group containing from 1 to 3 carbon atom.

9. A process as claimed in claim 8 in which the lower alkyl group containing from 1 to 3 carbon atom is a methyl group.

10. A process as claimed in claims 8 and 9 in which the reaction is carried out in the presence of a polar inert organic solvent.

11. A process as claimed in claim 10 in which the polar inert organic solvent includes acetonitrile or dioxane.

12. A process as claimed in claims 8 to 11 in which the reaction is effected at room temperature.

13. Pharmaceutical compositions comprising as active ingredient at least one compound of general formula (I) as defined in claim 1 or tautomeric or physiologically compatible acid addition salts thereof, in association with a pharmaceutical carrier or excipient.

14. Pharmaceutical compositions as claimed in claim 13 for use as anti-ulcers.

15. Pharmaceutical compositions as claimed in claim 13 for treatment of patients suffering from disorders of gastrointestinal tract.

**Claims for the Contracting State: AT**

1. A process of the preparation of compounds of general formula (I)

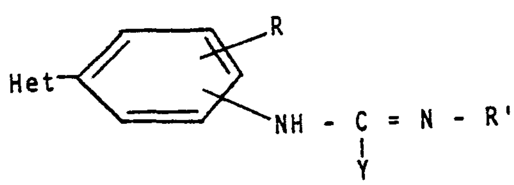

(I)

wherein Het represents an unsaturated five-membered heterocyclic ring containing from 2 to 3 nitrogen atoms, R is a hydrogen atom, a lower alkyl or alkoxy group containing from 1 to 3 carbon atoms or a halogen atom, Y represents a group of formula —$SO_3H$ or of formula

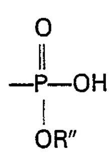

(where R″ is a lower alkyl group containing from 1 to 3 carbon atoms), R′ represents a linear or branched $C_1$—$C_8$ alkyl group which may contain a hetero atom such as sulfur, an oxygen or nitrogen atom, a linear or branched $C_3$—$C_5$ alkenyl group, a $C_3$—$C_7$ cycloalkyl or alkyl cycloalkyl group, phenyl group (optionally substituted by a lower alkyl or alkoxy group containing from 1 to 3 carbon atoms or a halogen atom), unsubstituted benzyl when Het is imidazole, R is hydrogen and Y is a sulphonic or phosphonic group; tautomers thereof and acid addition salts of the aforesaid compounds, which comprises reacting a compound of formula (II)

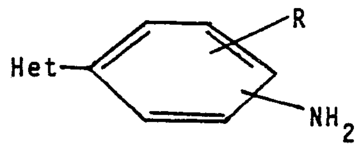

(II)

in which Het and R are as defined above with the compound of formula (III)

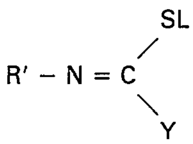

(III)

in which R′ and Y are as defined above and L represents a lower alkyl group containing from 1 to 3 carbon atom.

2. A process as claimed in claim 1 in which the lower alkyl group containing from 1 to 3 carbon atom is a methyl group.

3. A process as claimed in claim 2 in which the reaction is carried out in the presence of a polar inert organic solvent.

4. A process as claimed in claim 3 in which the polar inert organic solvent includes acetonitrile or dioxane.

5. A process as claimed in claim 1 in which the reaction is effected at room temperature.

**Patentansprüche für die Vertragsstaaten: BE CH DE FR GB IT LI LU NL SE**

1. Verbindungen der allgemeinen Formel (I)

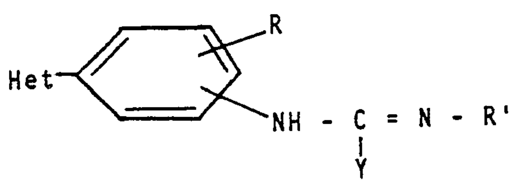

(I)

worin Het einen ungesättigten fünfgliedrigen heterozyklischen Ring mit 2 bis 3 Stickstoffatomen bedeutet, R Wasserstoff, eine niedrige Alkyl- oder Alkoxygruppe mit 1 bis 3 Kohlenstoffatomen oder ein Halogen bedeutet, Y eine Gruppe der Formel —$SO_3H$ oder der Formel

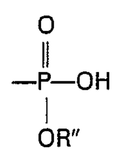

(worin R″ eine niedrige Alkylgruppe mit 1 bis 3 Kohlenstoffatomen bedeutet) bedeutet, R′ bedeutet eine lineare oder verzweigte $C_1$—$C_8$ Alkylgruppe, die ein Heteroatom wie Schwefel, Sauerstoff oder Stickstoff enthalten kann, eine lineare oder verzweigte $C_3$—$C_5$ Alkenylgruppe, eine $C_3$—$C_7$ Zykloalkyl- oder Alkyl-Zykloalkylgruppe, eine Phenylgruppe (gegebenenfalls substituiert durch eine niedrige Alkyl- oder Alkoxygruppe mit 1 bis 3 Kohlenstoffatomen oder durch ein Halogen), ein unsubstituiertes Benzyl, wenn Het Imidazol bedeutet, R bedeutet Wasserstoff und Y ist eine Sulphonium- oder Phosphoniumgruppe; deren Tautomere und Säureadditionssalze der zuvor enwähnten Verbindungen.

2. Physiologisch verträgliche Säureadditionssalze der Verbindungen der Formel (I) nach Anspruch 1.

3. Salze nach Anspruch 2, die mit Salzsäure, Maleinsäure, Schwefelsäure oder Fumarsäure gebildet werden.

4. Verbindungen nach einem der vorangehenden Ansprüche, dadurch gekennzeichnet, daß sich das Sulphonyl oder Phosphonylamidin Radikal in der para-Position des Benzolringes bezüglich der Het-Gruppe befindet.

5. Verbindungen nach einem der vorangehenden Ansprüche, dadurch gekennzeichnet, daß Het eine 2-Imidazolyl-, 4-Imidazolyl, 3-Pyrazolyl- oder 3-(1,2,4-Triazolyl)-Gruppe bedeutet, R Wasserstoff, eine Methoxygruppe oder Chlor bedeutet, Y eine Gruppe der Formel $-SO_3H$ oder der Formel

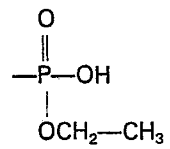

bedeutet, R' eine lineare oder verzweigte Alkyl- oder Alkenylgruppe mit 3 bis 5 Kohlenstoffatomen bedeutet (gegebenenfalls mit einem Schwefel oder einem Sauerstoffatomen oder einem Stickstoff in einer Cyanogruppe).

6. Physiologisch verträgliche Säureadditionssalze der Verbindungen nach Anspruch 5.

7. Maleinsäure-, Salzsäure, Schwefelsäure- oder Fumarsäure-Additionssalze der Verbindungen nach Anspruch 5.

8. Verfahren zur Herstellung der Verbindungen der allgemeinen Formel (I) nach Anspruch 1, welches die Reaktion einer Verbindung der Formel (II)

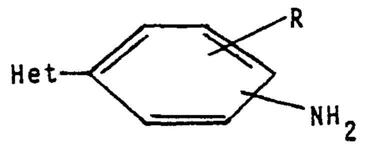

(II)

worin Het und R wie in Anspruch 1 definiert sind, mit einer Verbindung der Formel (III)

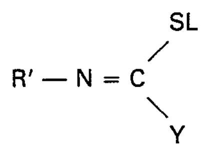

(III)

worin R' und Y wie in Anspruch 1 definiert sind und L eine niedrige Alkylgruppe mit 1 bis 3 Kohlenstoffatomen bedeutet, umfaßt.

9. Verfahren nach Anspruch 8, dadurch gekennseichnet, daß die niedrige Alkylgruppe mit 1 bis 3 Kohlenstoffatomen eine Methylgruppe ist.

10. Verfahren nach den Ansprüchen 8 und 9, dadurch gekennzeichnet, daß die Reaktion in Gegenwart eines polaren inerten organischen Lösungsmittels durchgeführt wird.

11. Verfahren nach Anspruch 10, dadurch gekennzeichnet, daß das polare inerte organische Lösungsmittel Acetonitril oder Dioxan einschließt.

12. Verfahren nach den Ansprüchen 8 bis 11, dadurch gekennzeichnet, daß die Reaktion bei Raumtemperatur durchgeführt wird.

13. Pharmazeutische Zusammensetzungen, die als wirksamen Bestandteil mindestens eine Verbindung der allgemeinen Formel (I) nach Anspruch 1 oder dessen tautomeren oder physiologisch verträgliche Säureadditionssalze in Verbindung mit einem pharmazeutischen Trägermaterial oder Exzipienten enthalten.

14. Pharmazeutische Zusammensetzungen nach Anspruch 13 zur Verwendung als Antiulcuspräparate.

15. Pharmazeutische Zusammensetzungen nach Anspruch 13 zur Behandlung von Patienten, dei an Störungen des gastrointestinal Traktes leiden.

**Patentansprüche für den Vertragsstaat: AT**

1. Verfahren zur Herstellung von Verbindungen der allgemeinen Formel (I):

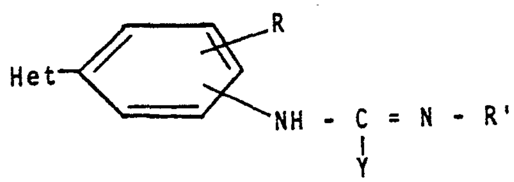

(I)

worin Het einen ungesättigten fünfgliedrigen heterozyklischen Ring mit 2 bis 3 Stickstoffatomen bedeutet, R Wasserstoff, eine niedrige Alkyl- oder Alkoxygruppe mit 1 bis 3 Kohlenstoffatomen oder ein Halogen bedeutet, Y eine Gruppe der Formel $-SO_3H$ oder der Formel

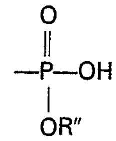

(worin R″ eine niedrige Alkylgruppe mit 1 bis 3 Kohlenstoffatomen ist) bedeutet, R′ eine lineare oder verzweigte $C_1$—$C_8$ Alkylgruppe bedeutet, die ein Heteroatomen wie Schwefel, Sauerstoff oder Stickstoff enthalten kann, eine lineare oder verzweigte $C_3$—$C_5$ Alkenylgruppe oder eine $C_3$—$C_7$ Zykloalkyl- oder Alkyl-Zykloalkylgruppe, eine Phenylgruppe (gegenbenenfalls durch eine niedrige Alkyl- oder Alkoxygruppe mit 1 bis 3 Kohlenstoffatomen oder ein Halogen substituiert), ein unsubstituiertes Benzyl wenn Het Imidazol bedeutet, R Wasserstoff bedeutet, und Y eine Sulphonium- oder Phosphoniumgruppe bedeutet, deren Tautomere und die Säureadditionssalze der zuvor erwähnten Verbindungen; welches die Reaktion einer Verbindung der Formel (II)

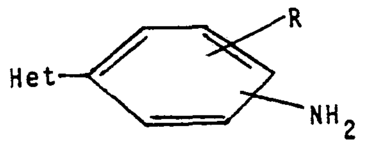

(II)

worin Het und R wie oben definiert sind, mit einer Verbindung der Formel (III)

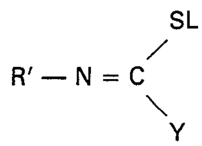

(III)

worin R′ und Y wie oben definiert sind, und L eine niedrige Alkylgruppe mit 1 bis 3 Kohlenstoffatomen bedeutet, umfaßt.

2. Verfahren nach Anspruch 1, dadurch gekennzeichnet, daß die niedrige Alkylgruppe mit 1 bis 3 Kohlenstoffatomen eine Methylgruppe ist.

3. Verfahren nach Anspruch 1, dadurch gekennzeichnet, daß die Reaktion in Gegenwart eines polaren inerten organischen Lösungsmittel durchgeführt wird.

4. Verfahren nach Anspruch 3, dadurch gekennzeichnet, daß das polare inerte organische Lösungsmittel Acetonitril oder Dioxan einschließt.

5. Verfahren nach Anspruch 1, dadurch gekennzeichnet, daß die Reaktion bei Raumtemperatur durchgeführt wird.

**Revendications pour les Etats contractants: BE CH DE FR GB IT LI LU NL SE**

1. Composés de formule générale (I)

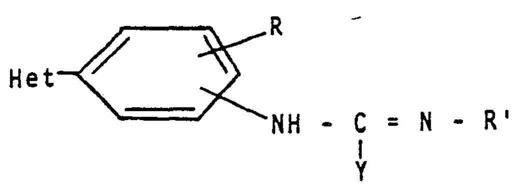

(I)

dans laquelle Het représente un noyau hétérocyclique insaturé à 5 chaînons contenant de 2 à 3 atomes d'azote, R est un atome d'hydrogène, un groupe alkyle ou alcoxy en $C_1$—$C_3$ ou un atome d'halogène, Y représente un groupe de formule —$SO_3H$ ou de formule

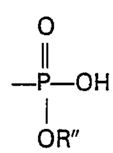

(dans laquelle R″ est un groupe alkyle inférieur en $C_1$—$C_3$), R′ représente un groupe alkyle linéaire ou ramifié en $C_1$—$C_8$ qui peut contenir un hétéroatome tel qu'un atome de soufre, d'oxygène ou d'azote, un groupe alcényle linéaire ou ramifié en $C_3$—$C_5$, un groupe cycloalkyle en $C_3$—$C_7$ ou alkylcycloalkyle, un groupe phényle (facultativement substitué par un groupe alkyle ou alcoxy en $C_1$—$C_3$ ou un atome d'halogène), un groupe benzyle non substitué lorsque Het est l'imidazole, R est l'hydrogène et Y est un

groupe sulfonique ou phosphonique; leurs tautomères et les sels d'addition d'acides des composés ci-dessus.

2. Sels d'addition d'acides physiologiquement compatibles des composés de formule (I) selon la revendication 1.

3. Sels selon la revendication 2, formés ave l'acide chlorhydrique, l'acide maléique, l'acide sulfurique ou l'acide fumarique.

4. Composés selon l'une quelconque des revendications précédentes, dans lesquels le radical sulfonyle ou phosphonylamidine est en position para du noyau benzénique par rapport au groupe Het.

5. Composés selon l'une quelconque des revendications précédentes, dans lesquels Het représente un groupe 2-imidazolyle, 4-imidazolyle, 3-pyrazolyle ou 3-(1,2,4-triazolyle), R représente un atome d'hydrogène, un groupe méthyle ou méthoxy ou un atome de chlore, Y est un groupe de formule —$SO_3H$ ou de formule

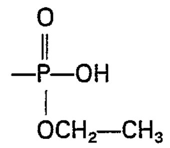
,

R' représente un groupe alkyle ou alcényle linéaire ou ramifié en $C_3$—$C_5$ (contenant facultativement un atome de soufre ou d'oxygène ou un atome d'azote dans un groupe cyano).

6. Sels d'addition d'acide physiologiquement compatibles des composés selon la revendication 5.

7. Sels d'addition d'acide maléique, chlorhydrique, sulfurique ou fumarique des composés selon la revendication 5.

8. Procédé pour la préparation des composés de formule générale (I) selon la revendication 1, qui comprend la rèaction d'un composé de formule (II)

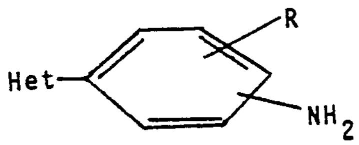
(II)

dans laquelle Het et R sont définis comme à la revendication 1, avec un composé de formule (III)

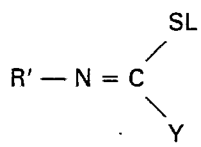
(III)

dans laquelle R' et Y sond définis comme à la revendication 1 et L représente un groupe alkyle inférieur en $C_1$—$C_3$.

9. Procédé selon la revendication 8, dans lequel le groupe alkyle inférieur en $C_1$—$C_3$ est un groupe méthyle.

10. Procédé selon les revendications 8 et 9, dans lequel la réaction est effectuée en présence d'un solvant organique inerte polaire.

11. Procédé selon la revendication 10, dans lequel le solvant organique inerte polaire contient de l'acétonitrile ou du dioxanne.

12. Procédé selon les revendications 8 à 11, dans lequel la réaction est effectuée à la température ambiante.

13. Compositions pharmaceutiques comprenant comme ingrédient actif au moins un composé de formule générale (I) tel que défini à la revendication 1 ou un de ses tautomères ou sels d'addition d'acide physiologiquement compatibles, en association avec un véhicule ou récipient pharmaceutique.

14. Compositions pharmaceutiques selon la revendication 13 pour l'utilisation comme anti-ulcères.

15. Compositions pharmaceutiques selon la revendication 13 pour le traitement de patients souffrant de troubles du tube gastro-intestinal.

**Revendications pour l'Etat contractant: AT**

1. Procédé pour la préparation de composés de formule générale (I)

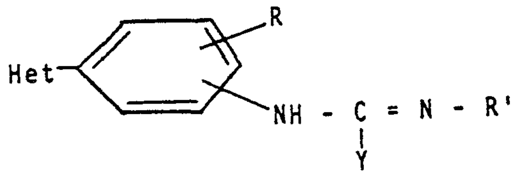

(I)

dans laquelle Het Het représente un noyau hétérocyclique insaturé à 5 chainons contenant de 2 à 3 atomes d'azote, R est un atome d'hydrogène, un groupe alkyle ou alcoxy en $C_1$—$C_3$ ou un atome d'halogène, Y représente un groupe de formule —$SO_3H$ ou de formule

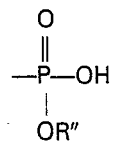

(dans laquelle R″ est un groupe alkyle inférieur en $C_1$—$C_3$), R′ représente un groupe alkyle linéaire ou ramifié en $C_1$—$C_8$ qui peut contenir un hétéroatome tel qu'un atome de soufre, d'oxygène ou d'azote, un groupe alcényle linéaire ou ramifié en $C_3$—$C_5$, un groupe cycloalkyle en $C_3$—$C_7$ ou alkylcycloalkyle, un groupe phényle (facultativement substitué par un groupe alkyle ou alcoxy en $C_1$—$C_3$ ou un atome d'halogène), un groupe benzyle non substitué lorsque Het est l'imidazole, R est l'hydrogène et Y est un groupe sulfonique ou phosphonique; leurs tautomères et les sels d'addition d'acides des composés ci-dessus, qui comprend la réaction d'un composé de formule (II)

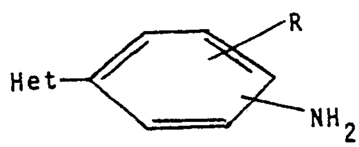

(II)

dans laquelle Het et R sont tels que définis ci-dessus avec un composé de formule (III)

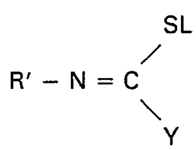

(III)

dans laquelle R′ et Y sont tels que définis ci-dessus et L représente un groupe alkyle inférieur en $C_1$—$C_3$.

2. Procédé selon la revendication 1, dans lequel le groupe alkyle inférieur en $C_1$—$C_3$ est un groupe méthyle.

3. Procédé selon la revendication 1, dans lequella réaction est effectuée en présence d'un solvant organique inerte polaire.

4. Procédé selon la revendication 3, dans lequel le solvant organique inerte polaire contient de l'acétonitrile ou du dioxanne.

5. Procédé selon la revendication 1, dans lequelle réaction est effectuée à la température ambiante.